**Europäisches Patentamt**

(19) **European Patent Office**

**Office européen des brevets**

(11) Veröffentlichungsnummer: **0 002 438**
**B1**

(12) **EUROPÄISCHE PATENTSCHRIFT**

(45) Veröffentlichungstag der Patentschrift: **01.04.81**

(21) Anmeldenummer: **78101065.7**

(22) Anmeldetag: **04.10.78**

(51) Int. Cl.³: **C 12 N 9/02, A 61 K 37/50**

(54) Lagerfähige Cholesterinoxidasepräparation.

(30) Priorität: **14.12.77 DE 2755799**

(43) Veröffentlichungstag der Anmeldung:
**27.06.79 Patentblatt 79/13**

(45) Bekanntmachung des Hinweises auf die Patenterteilung:
**01.04.81 Patentblatt 81/13**

(84) Benannte Vertragsstaaten:
**BE CH DE FR GB LU NL SE**

(56) Entgegenhaltungen:
**DE - A - 2 711 720**
**DE - A - 2 712 007**

(73) Patentinhaber: **BOEHRINGER MANNHEIM GMBH**
**Sandhofer Strasse 112-132 Postfach 31 01 20**
**D-6800 Mannheim 31-Waldhof (DE)**

(72) Erfinder: **Thum, Waldemar**
**Staudenmoosstrasse 5**
**D-8132 Tutzing-Unterzeismering (DE)**
Erfinder: **Lang, Gunter**
**Lange Strasse 22**
**D-8132 Tutzing (DE)**
Erfinder: **Vetter, Hellmuth, Dr.**
**Kreuzeckstrasse 17**
**D-8132 Tutzing (DE)**
Erfinder: **Näher, Gotthilf, Dr.**
**Zugspitzstrasse 22**
**D-8132 Tutzing (DE)**

(74) Vertreter: **Weickmann, Heinrich, Dipl.-Ing. et al,**
**Postfach 860 820 Möhlstrasse 22**
**D-8000 München 86 (DE)**

Courier Press, Leamington Spa, England.

# 0 002 438

Lagerfähige Cholesterinoxidasepräparation

Die Erfindung betrifft eine lagerfähige, wäßrige Cholesterinoxidasepräparation mit einem Gehalt an Puffer und an Stabilisierungsmittel.

Die enzymatische Aktivität eines Proteins wird von der räumlichen Anordnung der Aminosäurereste in seinen Polypeptidketten bestimmt. Diese "Konformation" wird in wäßriger Lösung durch die Anwesenheit von elektrisch geladenen oder ungeladenen anorganischen wie organischen Molekülen beeinflußt. Infolgedessen sind Enzyme in der Regel in wäßriger Lösung sehr empfindlich und erleiden leicht einen Aktivitätsverlust. Wäßrige Enzympräparationen müssen daher stabilisiert werden.

Hinsichtlich der Wirkung von niedermolekularen anorganischen Salzen ist bekannt, daß deren anionische wie kationische Komponenten entweder mehr stabilisierend oder mehr denaturierend auf die Konformation des Enzyms einwirken können. Derartige Anionen und Kationen sind hinsichtlich dieser Wirkung in der sogenannten Hoffmeister-reihe klassifiziert ("Structure and Stability of biological Macromolecules" von Timasheff und Fasman, Verlag Mercel Dekker, Inc. New York, 1969, Seite 427.) Nach der Hoffmeister-reihe sind Anionen und Kationen jeweils unabhängig entweder mehr stabilisierend oder mehr denaturierend wirksam. An der Spitze der Stabilisierungswirkung stehen dabei das Sulfatanion und quarternäre Ammoniumionen, gefolgt von Ammoniumion. Es ist daher leicht erklärlich, daß die überwiegende Zahl der handelsüblichen Enzympräparate als Stabilisierungsmittel Ammoniumsulfat enthält, allein oder gegebenenfalls mit zusätzlichen spezifischen Stabilisierungsmitteln. Auch die Cholesterinoxidase ist in handelsüblicher wäßriger Lösung bisher mit Ammoniumsulfat stabilisiert worden.

Es hat sich jedoch gezeigt, daß die Stabilität von Cholesterinoxidase in Ammoniumsulfatlösung zu Beanstandungen Anlaß gibt. Die beste Stabilisierungswirkung wurde bei etwa 1 M Ammoniumsulfat erzielt und nimmt bei höherer Ammoniumsulfatkonzentration ab. Bereits bei einer Konzentration von 1 M treten jedoch gelegentlich grobflokkige fettige Ausfällungen des Enzyms auf, die zwar immer noch enzymatisch aktiv sind, jedoch die Dosierung bei der Abfüllung beeinträchtigen.

Überraschenderweise wurde nunmehr gefunden, daß diese Schwierigkeit beseitig werden kann und einerseits Ausfällungen völlig zu vermeiden sind und andererseits eine überlegene Stabilisierung erzielt wird, wenn man als Stabilisierungsmittel Natriumchlorid oder Kaliumchlorid in einer Konzentration zwischen 2,0 und 3,5 M anwendet.

Gegenstand der Erfindung ist daher eine lagerfähige wäßrige Cholesterinoxidasepräparation mit einem Gehalt an Puffer und an Stabilisierungsmittel, die dadurch gekennzeichnet ist, daß sie pro Liter 2 bis 3,5 Mole NaCl oder KCl als Stabilisierungsmittel enthält. Vorzugsweise enthält sie NaCl in einer Konzentration zwischen 2,5 und 3,2 M.

Die überlegene Stabilisierungswirkung der genannten Salze war nicht zu erwarten, da nach der Hoffmeister-reihe Kalium und Natrium beträchtlich weniger stabilisieren, d.h. stärker inaktivieren als Ammonium und auch Chlorid erheblich stärker inaktiviert als Sulfat. Hinzu kommt noch, daß bekanntlich Flavinenzyme, zu denen auch die Cholesterinoxidase gehört, durch Alkalichloride bei höherer Konzentration inaktiviert werden (Theorell, in Acta Chem. Scand. 3, 1954, S. 1649).

Unter den vorstehend angegebenen Bedingungen liegt gute Stabilität bei pH-Werten zwischen 4,5 und 8,5 vor. Vorzugsweise besitzt die erfindungsgemäße Cholesterinoxidasepräparation einen pH-Wert zwischen 5,0 und 6,5. Die Puffermolarität liegt im allgemeinen zwischen 0,01 und 0,2 M, bevorzugt wird der Bereich von 0,01 M bis 0,1 M.

Als Puffersubstanzen können alle im oben angegebenen Bereich puffernden Salze verwendet werden, beispielsweise Kaliumphosphatpuffer, Natriumphosphatpuffer, Kaliumphosphat / Zitronensäure-puffer, Natriumacetatpuffer usw.

Man hat zwar in Einzelfällen bereits Enzyme in Alkalichloridlösungen gelagert. Hierfür waren jedoch Verfahrensgründe maßgebend, beispielsweise die bessere Kristallisierfähigkeit von Papain in Natriumchlorid, verglichen mit Ammoniumchlorid oder die Reinigung der Lectine durch Chromatographie in Natriumchloridlösungen, so daß man eine verschlechterte Stabilität in Kauf nehmen konnte. Für Flavinenzyme war jedoch eine derartige Lagerungsform nicht bekannt, da sie zur Abspaltung der prostetischen Gruppe bei sauren pH-Werten führt.

Die überlegene Lagerungsstabilität der erfindungsgemäßen Cholesterinoxidasepräparate gegenüber mit Ammoniumsulfat stabilisierten Cholesterinoxidasepräparaten ergibt sich aus folgenden Versuchen:

Eine Charge Cholesterinoxidase aus Nocardia erythropolis (Boehringer Mannheim Nr. 120 812) mit einem Gehalt von 450 U/ml Enzym in 0,05 M Kaliumphosphatpuffer, pH 6, wurde auf die in der nachstehenden Tabelle aufgeführte Salzkonzentration eingestellt und 4 Wochen bei 4°C bzw. +35°C gehalten. Die nach Ablauf dieser Zeit festgestellte Restaktivität zeigt nachstehende Tabelle (2-Wochenwerte in Klammern).

## 0 002 438

### TABELLE I

| Belastungstemperatur | Art und Konzentration der Zusätze | | |
|---|---|---|---|
| | 1 M AS | 3 M NaCl | 3 M KCl |
| + 4°C | (100%) 100% trüb | (96%) 96% blank | (100%) 100% blank |
| + 33°C | (86%) 55% trüb | (88%) 88% blank | (91%) 87% blank |

AS = Ammoniumsulfat

Die überlegene Stabilisation gemäß Erfindung wird unabhängig von der Enzymkonzentration und von der Enzymcharge (einzelne Ansatzchargen unterscheiden sich vielfach in ihrer Stabilität) erhalten und ist noch stärker ausgeprägt, wenn nicht mit 1 M Ammoniumsulfat, sondern mit 3 M Ammoniumsulfat-Lösung verglichen wird.

Es wurden zwei verschiedene Enzymchargen auf eine Konzentration von 300 U/ml bzw. 30 U/ml mit dem oben angegebenen Puffer eingestellt und dann wie oben beschrieben 4 Wochen auf 35°C gehalten. Nachstehende Tabelle gibt die nach 4 Wochen gemessenen Restaktivitäten an. In Klammern sind die 2-Wochenwerte angegeben.

### TABELLE II

| Charge | Enzymkonzentration | 1 M AS | 3 M AS | 3 M NaCl |
|---|---|---|---|---|
| 1 | 300 U/ml | (73%) 58% | | (100%) 92% |
| | 30 U/ml | (77%) 67% | (68%) 49% | (100%) 96% |
| 2 | 300 U/ml | (73%) 68% | | (100%) 88% |
| | 30 U/ml | (55%) 55% | (87%) 33% | (100%) 88% |

Um zu zeigen, daß die überlegene Stabilisierung gemäß Erfindung auf einen bestimmten Konzentrationsbereich beschränkt ist, wurde bei einer Enzymcharge mit einer Konzentration von 30 U/ml der oben beschriebene Alterungstest bei 4°C bzw. 35°C 4 Wochen (2 Wochen) mit zwei verschiedenen Ammoniumsulfat- und vier verschiedenen Natriumchlorid-konzentrationen durchgeführt. Die Ergebnisse zeigt Tabelle III.

### TABELLE III

| Temp. | Ammoniumsulfat | | Natriumchlorid | | | |
|---|---|---|---|---|---|---|
| | 1 M | 3,2 M | 2 M | 3 M | 4 M | 5 M |
| + 4°C | (95%) 100% | (100%) 90% | (72%) 95% | (64%) 95% | (76%) 80% | (100%) 80% |
| +35°C | (72%) 46% | (61%) 45% | (88%) 71% | (88%) 77% | (26%) 40% | (16%) 0 |

Die hier verwendete Enzymeinheit U/ml entspricht der Definition in Bergmeyer, Methoden der enzymatischen Analyse, Verlag Chemie Weinheim, 2. Auflage 1970, S. 276/277.

3

**Patentansprüche**

1. Lagerfähige, wäßrige Cholesterinoxidasepräparation mit einem Gehalt an Puffer und an Stabilisierungsmittel, dadurch gekennzeichnet, daß sie pro Liter 2 bis 3,5 Mole NaCl oder KCl als Stabilisierungsmittel enthält.

2. Präparation nach Anspruch 1, dadurch gekennzeichnet, daß sie pro Liter 2,5 bis 3,2 Mole NaCl enthält.

3. Präparation nach Anspruch 1 oder 2, dadurch gekennzeichnet, daß sie einen pH-Wert zwischen 4,5 und 8,5 aufweist.

4. Präparation nach Anspruch 3, dadurch gekennzeichnet, daß sie einen pH-Wert zwischen 5 und 6,5 aufweist.

5. Präparation nach einem der vorhergehenden Ansprüche, gekennzeichnet durch eine Puffersalzkonzentration von 0,01 bis 0,5 M.

**Revendications**

1. Préparation aqueuse, stable à la conservation, de cholestérol-oxydase avec une teneur en tampon et en stabilisant, caractérisée en ce qu'elle contient par litre 2 à 3,5 moles de NaCl ou KCl en tant que stabilisant.

2. Préparation selon la revendication 1, caractérisée en ce qu'elle contient par litre 2,5 à 3,2 moles de NaCl.

3. Préparation selon la revendication 1 ou 2, caractérisée en ce qu'elle présente une valeur de pH comprise entre 4,5 et 8,5.

4. Préparation selon la revendication 3, caractérisée en ce qu'elle présente une valeur de pH comprise entre 5 et 6,5.

5. Préparation selon l'une des revendications précédentes, caractérisée par une concentration en sel tampon de 0,01 à 0,5 M.

**Claims**

1. Storable, aqueous cholesterol oxidase preparation with a content of buffer and of stabilising agent, characterised in that, per litre, it contains 2 to 3.5 mole NaCl or KCl as stabilising agent.

2. Preparation according to claim 1, characterised in that, per litre, it contains 2.5 to 3.2 mole NaCl.

3. Preparation according to claim 1 or 2, characterised in that it has a pH value between 4.5 and 8.5.

4. Preparation according to claim 3, characterised in that it has a pH value between 5 and 6.5.

5. Preparation according to one of the preceding claims, characterised by a buffer salt concentration of 0.01 to 0.5M.